# FASCICULE DE BREVET EUROPEEN

(11) **EP 3 413 724 B1**
(45) Date de publication et mention de la délivrance du brevet: **20.05.2026**
(21) Numéro de dépôt: 17704740.4
(22) Date de dépôt: 10.02.2017
(51) Int. Cl.: A23K 10/18, A23L 33/135, C12R 1/25, C12R 1/245, A23K 50/10, A23K 50/40, A23K 50/60, A23K 50/80, C12N 1/20

(54) **UTILISATION D'UNE COMPOSITION PROBIOTIQUE POUR FAVORISER LA CROISSANCE JUVÉNILE DE L'ANIMAL D'ÉLEVAGE**
VERWENDUNG EINER PROBIOTISCHEN ZUSAMMENSETZUNG ZUR FÖRDERUNG DES WACHSTUMS VON JUNGTIEREN
USE OF A PROBIOTIC COMPOSITION FOR PROMOTING JUVENILE LIFESTOCK GROWTH

(30) Priorité: 12.02.2016 FR 1651170
(43) Date de publication de la demande: 19.12.2018
(73) Titulaire: Ecole Normale Supérieure de Lyon, 69007 Lyon (FR); Centre National de la Recherche Scientifique, 75016 Paris (FR); Université Claude Bernard Lyon 1, 69100 Villeurbanne (FR)
(72) Inventeur: LEULIER, François, 69360 Serezin du Rhone (FR); SCHWARZER, Martin, 69360 Solaize (FR)
(74) Mandataire: Regimbeau
(86) Numéro de dépôt international: PCT/EP2017/052982
(87) Numéro de publication internationale: WO 2017/137547

(56) Documents cités:
- EP-B1- 2 768 312
- WO-A1-2015/173386
- FR-A3- 2 877 811
- J. BAAH ET AL: "Impact of a mixed culture of Lactobacillus casei and L. lactis on in vitro ruminal fermentation and the growth of feedlot steers fed barley-based diets", CANADIAN VETERINARY JOURNAL - REVUE VETERINAIRE CANADIENNE, vol. 89, no. 2, 1 June 2009 (2009-06-01), CA, pages 263 - 271, XP055282481, ISSN: 0008-5286, DOI: 10.4141/CJAS08117
- AGUSTINA R ET AL: "Probiotics Lactobacillus reuteri DSM 17938 and Lactobacillus casei CRL 431 modestly increase growth, but not iron and zinc status, among Indonesian children aged 1-6 years", THE JOURNAL OF NUTRITION, AMERICAN SOCIETY FOR NUTRITION, US, vol. 143, no. 7, 1 July 2013 (2013-07-01), pages 1184 - 1193, XP002735351, ISSN: 0022-3166, [retrieved on 20130522], DOI: 10.3945/JN.112.166397
- SON V M ET AL: "Dietary administration of the probiotic, Lactobacillus plantarum, enhanced the growth, innate immune responses, and disease resistance of the grouper Epinephelus coioides", FISH AND SHELLFISH IMMUNOLOGY, ACADEMIC PRESS, LONDON, GB, vol. 26, no. 5, 1 May 2009 (2009-05-01), pages 691 - 698, XP026097888, ISSN: 1050-4648, [retrieved on 20090303], DOI: 10.1016/J.FSI.2009.02.018
- WANG J ET AL: "Lactobacillus plantarum ZLP001: In vitro Assessment of Antioxidant Capacity and Effect on Growth Performance and Antioxidant Status in Weaning Piglets", ASIAN-AUSTRALASIAN JOURNAL OF ANIMAL SCIENCES, SUWEON, KR, vol. 25, no. 8, 1 August 2012 (2012-08-01), pages 1153 - 1158, XP002735352, ISSN: 1011-2367, DOI: 10.5713/AJAS.2012.12079
- MARION BERNARDEAU ET AL: "Safety and efficacy of probiotic lactobacilli in promoting growth in post-weaning Swiss mice", INTERNATIONAL JOURNAL OF FOOD MICROBIOLOGY, vol. 77, no. 1-2, 1 July 2002 (2002-07-01), NL, pages 19 - 27, XP055282488, ISSN: 0168-1605, DOI: 10.1016/S0168-1605(02)00059-4
- VINCENZO CHIOFALO ET AL: "Effects of the administration of Lactobacilli on body growth and on the metabolic profile in growing Maltese goat kids", REPRODUCTION NUTRITION DEVELOPMENT, vol. 44, no. 5, 1 September 2004 (2004-09-01), pages 449 - 457, XP055282504, ISSN: 0926-5287, DOI: 10.1051/rnd:2004051

## Description

La présente invention concerne l'utilisation d'une composition probiotique permettant de favoriser la croissance juvénile des animaux vertébrés d'élevage. Elle concerne également l'utilisation d'une composition probiotique permettant d'augmenter le taux d'IGF-1 des animaux vertébrés d'élevage. Cette composition comprend comme principe actif ou ingrédient une souche *Lactobacillus plantarum* WJL, à tropisme intestinal.

Décrit comme « un organe additionnel », la communauté microbienne intestinale (ou microbiote intestinal) joue un rôle clé bénéfique pour l'hôte en exerçant de nombreuses fonctions biologiques, telles que l'aide à l'efficacité de la digestion, le métabolisme des substrats, la lutte contre les pathogènes, ou encore la mise en place et l'homéostasie des réponses immunitaires.

Définis en 2001 par l'Organisation mondiale de la Santé (OMS) et l'Organisation des Nations Unies pour l'alimentation et l'agriculture (FAO), les probiotiques sont des «micro-organismes vivants, qui, lorsqu'ils sont ingérés en quantité suffisante, exercent des effets positifs sur la santé, au-delà des effets nutritionnels traditionnels ».

WO2015173386 concerne une composition de Lactobacillus permettant de favoriser la croissance juvénile humaine et animale en cas de sous-nutrition. Toutefois dans ce contexte nutritionnel carencé cette composition, bien que permettant d'améliorer la croissance juvénile, ne permet pas à elle seule de restaurer entièrement une croissance optimale chez les sujets en sous-nutrition traités par cette composition. A contrario, on ne s'attend pas à ce qu'une composition de Lactobacillus ait un effet significatif sur la croissance juvénile d'animaux sous nutrition conventionnelle.

Certains acteurs de l'élevage ont recours à l'administration d'hormone de croissance ou d'antibiotiques pour favoriser la croissance des animaux d'élevage. Des travaux ont également décrit l'administration de probiotiques pour accroître la prise de poids.

Il existe toujours un besoin d'une solution efficace permettant de favoriser la croissance des animaux d'élevage, ne recourant pas à l'administration d'hormone ou de facteur de croissance ou d'antibiotiques, et induisant une croissance squelettique.

Un objectif de l'invention est donc de proposer une solution probiotique à ce besoin, avec des compositions permettant de favoriser la croissance juvénile, à savoir croissance pondérale et linéaire, chez des animaux d'élevage soumis à une nutrition conventionnelle, en pré- et/ou post-sevrage, notamment en post-sevrage.

Un autre objectif de l'invention est de favoriser la production d'IGF-1.

Un autre objectif de l'invention est de fournir de telles compositions, basées sur l'emploi d'une souche *Lactobacillus plantarum* WJL ayant un tropisme intestinal.

Un autre objectif encore de l'invention est de fournir de telles compositions favorisant notamment la croissance linéaire (que l'on appelle aussi croissance squelettique, résultat d'une croissance osseuse), la prise de poids en termes de masse musculaire, l'augmentation de la masse maigre (essentiellement masse musculaire et masse squelettique) et/ou la prise de longueur des os des animaux.

Un autre objectif encore de l'invention est de fournir de telles compositions et méthodes permettant de supporter une allégation santé et/ou nutritionnelle en accord avec la législation en vigueur, notamment la législation européenne.

L'invention est basée sur le fait que certaines souches de bactéries ayant un tropisme intestinal chez une espèce animale ont un effet favorisant la croissance juvénile chez un sujet de cette même espèce ou d'une autre espèce qui est soumis à une nutrition d'élevage conventionnelle (animal nourri selon les pratiques d'élevage en vigueur apportant une ration pour une croissance équilibrée) pour l'espèce animale ou l'animal considéré. Il a pu ainsi être démontré que des souches bactériennes du genre *Lactobacillus* étaient capables de favoriser la croissance juvénile dans un modèle souris en régime nutritionnel d'élevage conventionnel pour la souris, et en outre un lien a pu être établi entre ces résultats de croissance chez la souris et une augmentation du titre sérique d'Insuline-like Growth Factor 1 (IGF-1) chez les souris traitées dans ces conditions avec ces bactéries.

L'invention concerne donc l'utilisation et l'administration d'une souche *Lactobacillus plantarum* WJL à tropisme intestinal, afin de favoriser la croissance juvénile des animaux vertébrés d'élevage, en particulier pour la croissance post-sevrage. L'optimisation de la croissance juvénile ou de la croissance post-sevrage peut se mesurer sur différents critères objectifs pris isolément ou associés. Ces critères objectifs comprennent notamment la croissance linéaire (taille du museau ou de la bouche à la base de la queue), la prise de poids, de préférence rapportée liée à la croissance linéaire et/ou à une augmentation de la masse musculaire (l'objectif étant de s'affranchir de la prise de poids par augmentation de la masse graisseuse), la masse maigre dans laquelle on fera entrer la masse musculaire et la masse squelettique, la longueur des os ou la longueur du corps pour les poissons, l'utilisation selon l'invention conduisant à une augmentation du, des ou de plusieurs des critères observés. Avantageusement, ces critères sont accompagnés d'une augmentation du titre sérique IGF-1, exemplifié ci-dessous chez la souris. On peut également ne retenir que le critère d'augmentation du titre sérique d'IGF-1, notamment une fois la corrélation faite avec les autres critères.

La présente invention a donc pour objet l'utilisation d'une composition probiotique ou un aliment pour animal, comprenant une souche *Lactobacillus plantarum* WJL favorisant la croissance juvénile chez un animal d'élevage, de avec stimulation de la croissance linéaire, et/ou du taux d'IGF-1.

La bactérie a un « tropisme intestinal », ce qui signifie que la bactérie a la capacité de passer la barrière gastrique, soit naturellement soit en étant administrée dans une formulation gastro-protégée, et est capable de persister dans l'intestin de manière à produire un effet favorisant la croissance juvénile.

Conformément à une caractéristique avantageuse de l'invention, la bactérie favorise la production d'IGF-1 chez l'animal qui est traité par la composition conforme à l'invention. Une augmentation du taux d'IGF-1 peut notamment être corrélée à une croissance juvénile stimulée (qualifiable par les critères de croissance susdits). L'invention concerne donc l'utilisation d'une composition probiotique permettant de favoriser ou d'augmenter la production d'IGF-1 chez l'animal juvénile qui est traité par la composition conforme à l'invention.

Sont également décrites ici d'autres souches bactériennes, notamment des souches de bactérie appartenant aux familles suivantes : Lactobacillaceae, Streptoccaceae, Enterococcaceae, Leuconostocaceae, Bifidobacteriaceae. Sont également décrites ici une ou des souches du genre *Lactobacillus,* en particulier de l'une des espèces suivantes, *Lactobacillus delbrueckii, Lactobacillus plantarum, Lactobacillus fermentum, Lactobacillus casei, Lactobacillus paracasei, Lactobacillus rhamnosus.*

Plus particulièrement, il s'agit de bactéries appartenant aux espèces *Lactobacillus plantarum, Lactobacillus fermentum, Lactobacillus casei, Lactobacillus paracasei, Lactobacillus rhamnosus.* Suivant une modalité, la souche est choisie parmi les espèces *Lactobacillus plantarum, Lactobacillus fermentum, Lactobacillus casei.*

L'invention a pour objet l'utilisation d'une composition probiotique comprenant une souche de *Lactobacillus plantarum* WJL, tropisme intestinal, visant à favoriser la croissance juvénile des animaux vertébrés d'élevage en nutrition d'élevage conventionnelle, avec stimulation de la croissance linéaire et/ou du taux d'IGF-1.

L'invention a aussi pour objet l'utilisation d'une composition probiotique comprenant une souche de *Lactobacillus plantarum* WJL, à tropisme intestinal, visant à augmenter le taux d'IGF-1 chez des animaux vertébrés d'élevage en nutrition d'élevage conventionnelle.

Il s'agit d'une bactérie de l'espèce *Lactobacillus plantarum,* la souche WJL ou la souche G821, déposée à la Collection Nationale de Culture de Microorganismes (Institut Pasteur) sous le numéro d'enregistrement CNCM I-4979 le 11 mai 2015. La souche G821 a été obtenue par évolution expérimentale (c.a.d. par accumulation et sélection de variants naturels) de la souche *L. plantarum* NIZO2877. Bien entendu, la composition selon l'invention peut comprendre plus d'une souche de bactérie répondant aux besoins de l'invention. Notamment, la composition comprend 2 ou plus de ces souches de bactéries, choisies dans une même espèce ou dans des espèces différentes.

La bactérie est un *L. plantarum* WJL (Eun-Kyoung Kim et al., Genome Announcements, November/December 2013, vol. 1, n° 6 e00937-13, GenBank AUTE00000000, *Lactobacillus plantarum* WJL, whole genome shotgun sequencing project). Cette souche WJL a été initialement isolée et peut être isolée de la drosophile (JH Ryu et al., Science 2008, 319 : 777-782).

D'autres exemples de souches appropriées sont les suivantes : *L. casei* ATCC 393, *L. casei* L919 (Koryszewska-Baginska A. et al., 26 septembre 2013, Genome Announc), *L. paracasei* ATCC25302, *L. paracasei* Shirota (Yuki N et al., Int J Food Microbiol. 1er avril 1999;48(1) :51-7), *L. fermentum* ATCC9338, *L. rhamnosus* L900 (Aleksandrzak-Piekarczyk T. et al., Genome Announc, 15 août 2013), *L. rhamnosus* L908 (Koryszewska-Baginska A. et al., 20 février 2014, Genome Announc), L. *rhamnosus* GG (Kankainen M. et al., Proc Natl Acad Sci USA, 6 octobre 2009).

La présente invention apporte donc à la technique l'enseignement que la souche *Lactobacillus plantarum* WJL à tropisme intestinal favorise la croissance juvénile d'animaux élevés en conditions de nutrition conventionnelles. Mais l'invention ne se limite pas à cet enseignement, elle donne en outre à l'homme du métier les outils lui permettant de déterminer de manière sûre les souches de bactéries utiles pour l'invention. Différents critères peuvent être à la base de tests, en étant utilisés seuls ou en association. Parmi ces critères, on notera le taux sérique d'IGF-1 chez l'animal modèle (e.g. souris), la croissance de souris modèles illustrée, par exemple, par la longueur des fémurs ou la croissance linéaire des animaux, ou encore la prise de poids, liée notamment à la croissance linéaire et/ou à l'augmentation de la masse musculaire et/ou à l'augmentation de la masse maigre. Sur la base de ces critères ou de critères similaires, il est possible pour l'homme du métier de mettre au point des tests comparant des individus élevés en présence ou en l'absence de la bactérie à tester.

On entend par organisme « axénique » un organisme (e.g. souris) élevé dans un environnement dépourvu de microorganismes et donc dépourvu de flore intestinale.

On entend par organisme « monoxénique » un organisme (e.g. souris) axénique associé élevé en présence d'un unique microorganisme et donc porteur de cet unique microorganisme en guise de flore intestinale.

Selon l'invention, il est possible de déterminer si une souche de bactérie à tropisme intestinal permet de favoriser la croissance en cas de nutrition conventionnelle, en utilisant un modèle souris axénique qui permet de réaliser un suivi de croissance linéaire des souris en présence de la bactérie à tester en comparaison avec l'absence de microbiote et/ou avec la présence d'une souche de bactérie de référence. Ce modèle peut être utilisé en première intention.

Suivant une caractéristique de l'invention, les souches de bactérie selon l'invention se caractérisent par le fait qu'elle répond positivement au test de croissance linéaire suivant :
- à partir d'une même lignée de souris (typiquement souris Balb/c) on établit une lignée de souris parentales axéniques et une lignée de souris parentales monoxéniques (associées à la bactérie à tester), et l'on produit des juvéniles qui sont élevées avec les parents en milieu nutritionnel conventionnel comprenant environ 40% d'hydrates de carbone, environ 25% de protéines et environ 9% de lipides, jusqu'à leur sevrage (à J21) ; pour former le groupe des juvéniles monoxéniques, on utilise des parents mono-associés avec la souche de bactérie à tester,
- à J21 : on dispose de 8 juvéniles sevrées issues de chacune de ces deux lignées, formant le groupe monoxénique et le groupe axénique, et on les élève en régime nutritionnel conventionnel comprenant environ 40% d'hydrates de carbone, environ 25% de protéines et environ 9% de lipides,
- à J 56 : on détermine la moyenne des tailles des souris pour un groupe considéré par la mesure de l'extrémité du museau à la base de la queue de chaque individu ; une autre mesure possible consiste à sacrifier les individus, à en prélever les fémurs et à mesurer la longueur de ces derniers,
- la souche de bactérie lactique étant considérée comme répondant positivement au test si la taille moyenne des individus et/ou la longueur moyenne des fémurs, du groupe monoxénique, est supérieur , respectivement, à la taille moyenne des individus et/ou à l longueur des fémurs, du groupe axénique, avec une valeur de p inférieure à 0,05, dans le test statistique de Tukey.

La présente invention a donc pour objet l'utilsation d'une composition comprenant une souche de *Lactobacillus plantarum* WJL ayant un tropisme intestinal, pour favoriser la croissance juvénile dans un contexte de nutrition conventionnelle, dans laquelle la souche de bactérie répond positivement au test de croissance linéaire sur souris. D'autres souches peuvent être identifiées parmi les bactéries à tropisme intestinal et notamment parmi les espèces et souches mentionnées plus haut, notamment parmi les souches *L. plantarum* G821, *L. casei* ATCC 393, *L. casei* L919, *L. paracasei* ATCC25302, *L. paracasei* Shirota, L. *fermentum* ATCC9338, *L. rhamnosus* L900, *L. rhamnosus* L908, *L. rhamnosus* GG.

Dans un mode de réalisation de l'invention, la souche WJL ou une autre souche à effet « marqué » est utilisée comme souche de référence afin d'identifier et de sélectionner des souches bactériennes ayant un effet « marqué » sur la croissance juvénile, à savoir un effet proche de celui de cette souche de référence, e.g. WJL (effet non significativement différent de la souche de référence, e.g. WJL), ou un effet « fort » sur la croissance juvénile (effet significativement supérieur à la souche de référence, e.g. WJL).

Pour ce faire, le test souris (incluant 8 souris par condition) est appliqué à la souche de référence, e.g. WJL et à la souche à tester (en parallèle, de préférence, ou alors on peut disposer de données de référence préalablement générées pour la souche WJL, par exemple les données présentées dans les exemples). On compare ensuite les moyennes obtenues pour les deux souches. La souche de bactérie testée est considérée comme étant une souche à effet marqué si la moyenne de la taille moyenne des individus et/ou de la longueur des fémurs du groupe monoxénique n'est pas significativement différente de la moyenne correspondante pour le groupe de référence, e.g. WJL avec une valeur de p supérieure à 0,05 dans le test statistique de Tukey. L'effet est fort si ladite moyenne pour la souche à tester est significativement supérieure à la moyenne pour la souche de référence, e.g. WJL, ce qui est le cas lorsque la valeur de p du test statistique est inférieure à 0,05. L'effet est qualifié d'intermédiaire si ladite moyenne pour la souche à tester (qui a été qualifiée par rapport aux souris axéniques dans le test précédent) est significativement inférieure à la moyenne pour la souche de référence, e.g. WJL, ce qui est le cas lorsque la valeur de p du test statistique est inférieure à 0,05.

La composition selon l'invention comprendra de préférence au moins une souche de bactérie ayant un tel effet marqué ou fort.

Les souches de bactérie selon l'invention peuvent se caractériser par le fait qu'elles ont un impact positif sur le taux d'IGF-1 sérique. C'est ainsi qu'il a été possible, sur la base d'un modèle de souris axéniques, de montrer que des souris élevées en milieu conventionnel et en présence de la bactérie (souris monoxéniques) avaient une croissance supérieure et en même temps un taux d'IGF-1 sérique supérieur, par rapport à ces mêmes souris élevées avec ce milieu conventionnel, mais en l'absence de la bactérie (souris axéniques). Ceci permet de proposer un test permettant de déterminer si une souche de bactérie a le potentiel à augmenter le taux sérique d'IGF-1, ce test pouvant être éventuellement appliqué en association avec un test de croissance linéaire afin de préciser ou d'affiner l'effet de la souche sur la croissance.

Dans ce cas, les souches de bactérie selon l'invention se caractérisent par le fait qu'elles répondent positivement au test du taux d'IGF-1 sérique suivant :
- à partir d'une même lignée de souris (typiquement souris Balb/c) on établit une lignée de souris parentales axéniques et une lignée de souris parentales monoxéniques (associées à la bactérie à tester), et l'on produit des juvéniles qui sont élevées avec les parents en milieu nutritionnel conventionnel environ 40% d'hydrates de carbone, environ 25% de protéines et environ 9% de lipides jusqu'à leur sevrage (à J21) ; pour former le groupe des juvéniles monoxéniques, on utilise des parents mono-associés avec la souche de bactérie à tester,
- à J21 : on dispose de 8 juvéniles sevrées issues de chacune de ces deux lignées, formant le groupe monoxénique et le groupe axénique, et on les élève en régime nutritionnel conventionnel comprenant environ 40% d'hydrates de carbone, environ 25% de protéines et environ 9% de lipides,
- à J 56 : on prélève du sang des juvéniles de chaque groupe et on détermine le taux sérique moyen d'IGF-1 pour chaque groupe; cette mesure du taux sérique d'IGF-1 est de préférence réalisée sur du sérum dilué (1:25) ; on utilise de préférence des kits ELISA commerciaux de détection de l'IGF-1 en suivant les instructions du fabricant,
- la souche de bactérie lactique est considérée comme répondant positivement au test si le taux sérique moyen d'IGF-1 du groupe monoxénique est supérieur au taux sérique moyen du groupe axénique avec une valeur de p inférieure à 0,05 dans le test statistique de Tukey.

La présente invention a donc pour objet l'utilisation d'une composition comprenant au moins une souche de *Lactobacillus plantarum* WJL, ayant un tropisme intestinal, pour favoriser la croissance juvénile sous nutrition conventionnelle, dans laquelle la souche de bactérie augmente le taux d'IGF-1 sérique. Il s'agit notamment d'une souche de bactérie qui répond positivement au test IGF-1 sur souris tel que décrit ci-dessus. D'autres souches peuvent être identifiées parmi les bactéries à tropisme intestinal et notamment parmi les espèces et souches mentionnées plus haut, notamment parmi les souches *L. plantarum* G821, *L. casei* ATCC 393, *L. casei* L919, *L. paracasei* ATCC25302, *L. paracasei* Shirota, *L. fermentum* ATCC9338, *L. rhamnosus* L900, *L. rhamnosus* L908, *L. rhamnosus* GG.

Dans un mode de réalisation de l'invention, la souche WJL ou une autre souche à effet « marqué» est utilisée comme souche de référence afin d'identifier et sélectionner des souches bactériennes ayant un effet « marqué » sur le taux sérique d'IGF-1, à savoir un effet proche de celui de cette souche de référence, e.g. WJL (effet non significativement différent de la souche de référence, e.g. WJL), ou un effet « fort » sur le taux sérique d'IGF-1 (effet significativement supérieur à la souche de référence, e.g. WJL).

Pour ce faire, le test souris (incluant 8 souris par condition) de mesure du taux sérique d'IGF-1 est appliqué à la souche de référence, e.g. WJL et à la souche à tester (en parallèle, de préférence, ou alors on peut disposer de données de référence préalablement générées pour la souche de référence, e.g. WJL, par exemple les données présentées dans les exemples). On compare ensuite les moyennes de taux sérique d'IGF-1 obtenues pour les deux souches. La souche de bactérie testée est considérée comme étant une souche à effet marqué si la moyenne des taux d'IGF-1 du groupe monoxénique n'est pas significativement différente de la moyenne pour le groupe de référence, e.g. WJL avec une valeur de p supérieure à 0,05, dans le test statistique de Tukey. L'effet est fort si ladite moyenne pour la souche à tester est significativement supérieure à la moyenne pour la souche de référence, e.g. WJL lorsque la valeur de p est inférieure à 0,05. L'effet est qualifié d'intermédiaire si ladite moyenne pour la souche à tester (préalablement qualifiée sur le test axénique précédent) est significativement inférieure à la moyenne pour la souche de référence, e.g. WJL lorsque la valeur de p est inférieure à 0,05.

La composition comprendra donc de préférence une souche de bactérie ayant un tel effet marqué ou fort, et en particulier cette souche de bactérie a un effet marqué ou fort à la fois sur la croissance linéaire et sur le taux sérique d'IGF-1.

La composition est utilisable chez les animaux d'élevage au sens large, comprenant notamment les animaux de pacage (bétail), les animaux de basse-cour, les animaux aquatiques, les animaux de compagnie. Notamment, la composition est utilisable sur les mammifères, notamment animaux de rente (bovins, ovins, caprins, porcins, aviaire), les animaux de compagnie (chien, chat) et de sport (cheval, dromadaire, chameau), de préférence entre le sevrage et la maturité sexuelle, ou entre le sevrage et le stade adulte (se caractérisant par l'atteinte de la taille adulte, fin de la croissance squelettique), ce que l'on appellera ici des animaux juvéniles. La composition peut être administrée à des animaux castrés, notamment avant la castration et/ou dans la période de croissance squelettique restante post-castration. Elle est aussi utilisable en élevage de poissons. Dans un mode de réalisation, l'animal est un carnivore. Dans un autre mode de réalisation, l'animal est un ruminant.

La composition peut notamment contenir une quantité d'environ 10⁵ à environ 10¹², notamment d'environ 10⁶ à environ 10¹², de préférence d'environ 10⁸ à environ 10¹² cellules bactériennes formant des colonies (CFU) selon l'invention, par gramme de composition. Le terme CFU signifie « colony forming units » selon l'expression technique anglaise. Par gramme de composition, on entend de préférence la composition probiotique formée des bactéries, co-ingrédients, et excipients ou vecteurs. Par cellules bactériennes, on entend une souche de bactérie unique conforme à l'invention ou un mélange d'au moins deux bactéries, conformes à l'invention.

La composition peut notamment comprendre la ou les bactéries lactiques sous forme vivante. La composition peut se présenter sous une forme prête à l'emploi ou à mélanger ou diluer avec un aliment, un excipient ou un liquide alimentaire tel que de l'eau de boisson.

Il peut s'agir d'une suspension bactérienne, qui peut être congelée et décongelée avant usage.

Il peut s'agir d'une poudre lyophilisée, pouvant être utilisée telle quelle, sous forme de poudre, de granulés, de comprimé, de bolus, de capsule, de gélule, ou utilisée après reprise dans un véhicule approprié. Cette composition peut comprendre un excipient de lyophilisation classique.

La composition peut être une forme d'administration orale (par exemple poudre, gélule, comprimé, bolus) sous une forme gastro-protégée permettant de passer l'estomac et libérer les bactéries dans l'intestin.

Suivant un mode de réalisation, la composition est une composition solide, e.g. comprimé, bolus, gélule, capsule, gastro-protégée permettant de passer l'estomac et libérer les bactéries dans l'intestin. Selon une modalité, la forme, notamment granulés, est utilisable en alimentation en milieu aqueux, pour les poissons notamment.

Suivant un mode de réalisation, la composition est un liquide ou est à dissoudre dans un liquide ou à mettre en suspension dans un liquide, par exemple l'eau de boisson, dans ce cas la composition est initialement solide, e.g. poudre, granulé, comprimé dissolvable, par exemple comprimé effervescent, ou comprimé déliquescent dans le liquide.

Suivant un mode de réalisation, la composition est solide, e.g. poudre, granulé, comprimé, bolus, et destinée ou apte à être mélangée à un aliment.

Suivant un mode de réalisation, la composition est solide, sous une forme prête à l'emploi sans besoin de mélange à un aliment, mais pouvant être néanmoins mélangée à un aliment, par exemple comprimé ou bolus appétant.

Est également divulgué un aliment pour élevage contenant au moins une bactérie ou composition selon l'invention. A titre d'exemple, l'aliment comprend la composition selon l'invention en mélange avec au moins des hydrates de carbone, des protéines et des lipides. L'aliment peut se présenter sous une forme liquide, émulsion ou solide, et parmi les formes solides on peut citer, à titre d'exemple, les granulés, bolus, flocons, ensilage additionné de la composition, fourrage additionné de la composition. L'aliment peut notamment être un aliment complet, un aliment fonctionnel, un aliment d'allaitement, un aliment formulé, un complément concentré.

Est également divulguée une méthode de traitement probiotique pour favoriser la croissance juvénile des animaux d'élevage, comprenant l'administration à un animal selon l'invention d'une composition selon l'invention. De préférence, la composition est administrée par voie orale. De préférence, la composition est administrée plusieurs fois dans une période allant du sevrage à la maturité sexuelle.

Est également divulguée une méthode d'élevage d'animaux, intégrant ce traitement probiotique.

Ce traitement probiotique ou méthode d'élevage comprend l'administration d'une quantité suffisante d'une composition telle que décrite ci-dessus au jeune animal, de préférence post-sevrage. La méthode comprendra l'administration en une ou plusieurs fois, pouvant être échelonnée sur la période de croissance du sujet (jusqu'à la maturité sexuelle), de doses de composition selon l'invention. Les doses peuvent être fractionnées pour faciliter l'administration. La fréquence d'administration est notamment comprise entre une dose (unique ou fractionnée) tous les jours et une dose tous les mois. Typiquement, la fréquence d'administration sera comprise entre une dose (unique ou fractionnée) tous les jours et une dose toutes les semaines, voire tous les 2, 3, 4, 5 ou 6 jours. Chaque dose (unique ou fractionnée) représente notamment plusieurs grammes à plusieurs dizaines de gramme de composition.

La méthode comprend notamment l'administration d'une composition comprenant une quantité d'environ 10⁵ à environ 10¹², notamment d'environ 10⁶ à environ 10¹², de préférence d'environ 10⁸ à environ 10¹² cellules bactériennes formant des colonies CFU par gramme de composition (par cellules bactériennes, on entend une souche de bactérie unique conforme à l'invention ou un mélange d'au moins deux bactéries, conformes à l'invention).

La méthode comprend de préférence l'administration d'une composition comprenant la ou les bactéries lactiques sous forme vivante.

La forme de la composition peut être solide ou liquide et prendre l'une quelconque des formes présentées *supra.*

Dans un mode de réalisation, la composition utilisée dans la méthode de traitement, comprend au moins une souche de bactérie qui n'est pas naturellement présente (« not-naturally occuring ») chez l'espèce traitée. Dans cette configuration, lorsqu'il y a plusieurs bactéries différentes, il suffit que l'une d'elles ne soit pas naturellement présente. En revanche, cette bactérie s'avère avoir un tropisme intestinal dans l'espèce animale et répond à la définition des souches actives selon l'invention.

L'invention concerne aussi une méthode de criblage de bactéries capables de favoriser la croissance juvénile sous nutrition conventionnelle, utilisant un modèle de souris axénique.

La méthode comprend les étapes suivantes :
- on dispose de juvéniles issues de deux lignées issues d'une même souche de souris (typiquement souris Balb/c), à savoir une lignée de souris parentales axéniques et une lignée de souris parentales monoxéniques (associées à la bactérie à tester),
- on les élève en régime nutritionnel conventionnel comprenant environ 40% d'hydrates de carbone, environ 25% de protéines et environ 9% de lipides,
- au terme d'une période d'élevage appropriée, on détermine la moyenne des valeurs d'un ou plusieurs paramètres de chaque groupe, ces paramètres étant liés à la croissance (par exemple prise de poids, croissance linéaire par exemple par mesure de la taille des individus ou de la longueur de leurs fémurs), et/ou au taux d'IGF-1 sérique,
- la souche de bactérie lactique est considérée comme répondant positivement au test si la valeur moyenne du paramètre mesuré du groupe monoxénique est supérieure à la valeur moyenne du paramètre mesuré du groupe axénique avec une valeur de p inférieure à 0,05 dans le test statistique de Tukey.

De préférence, la méthode de criblage reprend les caractéristiques du test souris croissance linéaire ou taux sérique d'IGF-1 décrit ci-dessus.

La méthode de criblage peut aussi être un test comparatif avec une souche de référence, par exemple la souche WJL, et ce test reprend alors les caractéristiques décrites ci-dessus pour les tests souris.

Les différents tests décrits peuvent tout à fait être réalisés avec un régime nutritionnel différent, pour autant qu'il convienne à une nutrition convenable des souris.

L'invention va être maintenant décrite plus en détail à l'aide de modes de réalisation de l'invention pris à titre d'exemples non limitatifs.

La descendance male (minimum 8 individus) de trois groupes d'individus issus d'une même colonie de souris axénique a été étudiée, le premier groupe consiste en des juvéniles axéniques (groupe Germ Free, GF), le deuxième en des juvéniles issus de parents mono-associés avec la souche *L. plantarum WJL* (groupe WJL), et le troisième en des juvéniles issus de parents mono-associés avec la souche *L. plantarum NIZO2877* (groupe NIZO 2877). Les parents et juvéniles sont élevés en milieu nutritionnel conventionnel (40% carbohydrates, 25,1% protéines, 9,1% lipides et 3646 kcal/kg) jusqu'au sevrage des juvéniles (Jour 21 post-naissance), puis les juvéniles sevrés sont élevés en milieu nutritionnel conventionnel jusqu'au Jour 56.

Quatre paramètres illustrant la croissance juvénile de ces individus ont été étudiés : le paramètre primaire étant la croissance linéaire ou prise de taille (mesure museau-base de la queue) pendant une période de 35 jours suivant le sevrage (des jours 21 à 56), puis trois paramètres secondaires que sont (1) la prise de poids pendant une période de 35 jours suivant le sevrage (des jours 21 à 56), (2) la longueur des fémurs d'un lot d'individus (au minimum 8 individus) représentatif de la population testée au jour 56 et enfin (4) le taux sérique au jour 56 du facteur de croissance IGF-1 chez au moins 8 individus.

Les analyses statistiques ont été réalisées en utilisant le test t en utilisant le logiciel GraphPad (GraphPad Prism 5.04, San Diego, USA) ; valeurs de p < 0.05 sont considérées significatives.

### (1) Prise de poids et de taille :

Les souris ont été anesthésiées par brève exposition à l'isoflurane pour permettre la mesure du poids et de la taille des souris (du museau à la base de la queue) à J21 et J56.

**Tableau 1: Prise de poids J21-J56 en g/jour**

| | GF | Lp WJL | Lp NIZO2877 |
|---|---|---|---|
| | 0,272286 | 0,357619 | 0,319337 |
| | 0,231714 | 0,369333 | 0,353623 |
| | 0,242857 | 0,353333 | 0,31648 |
| | 0,34 | 0,341905 | 0,291051 |
| | 0,263429 | 0,343333 | 0,283623 |
| | 0,311429 | 0,359048 | 0,250765 |
| | 0,331429 | 0,293333 | 0,276765 |
| | 0,322857 | 0,301905 | 0,385051 |
| | 0,308571 | | 0,345051 |
| | | | 0,345051 |
| | | | 0,373623 |
| | | | 0,327908 |
| Moyenne Erreur Standard | 0,291619111 | 0,339976125 | 0,322360667 |
| à la moyenne | 0,01328949 | 0,009779005 | 0,011782513 |
| Ecart-type | 0,03986847 | 0,027659203 | 0,040815822 |
| t-test pour séries non | | | |
| appariées | | | |
| Lp WJL | vs. | GF | p=0.0118 |
| Lp WJL | vs. | Lp NIZO2877 | p=0.3014 |
| Lp NIZO2877 | vs. | GF | p=0.1008 |

| | | | |
|---|---|---|---|
| Lp = L. plantarum | | | |

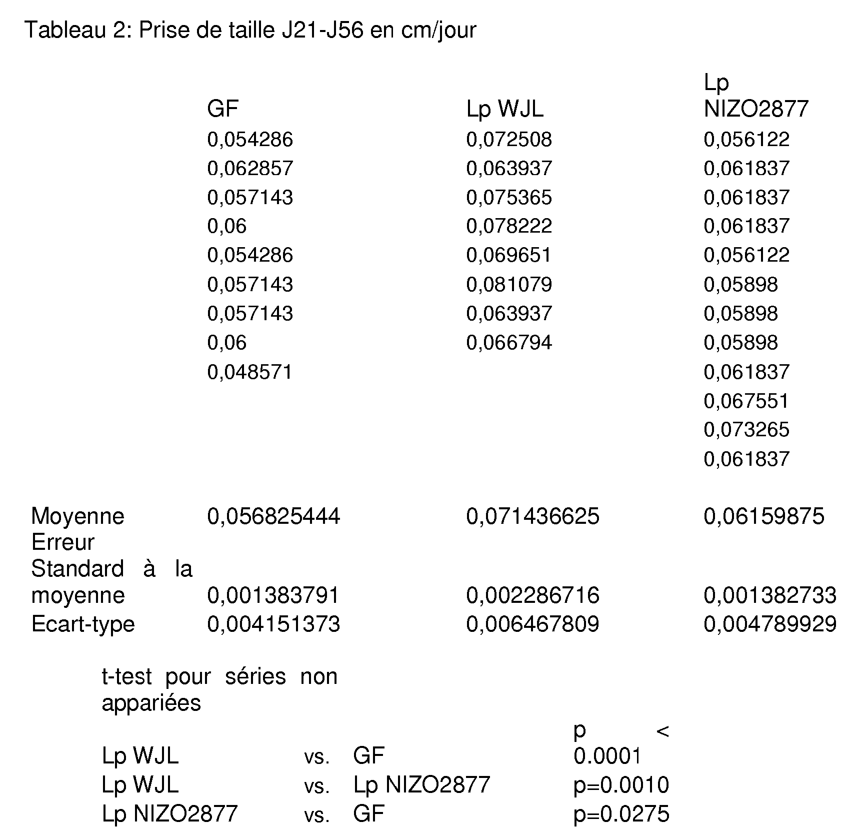

### (2) Longueur des fémurs :

Les souris sont sacrifiées à J56, un fémur est prélevé, libéré du muscle et sa longueur est mesurée au caliper Vernier.

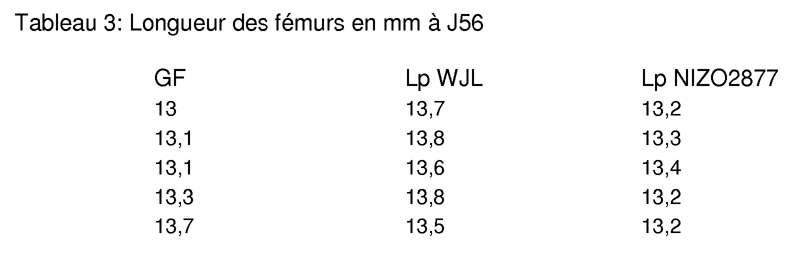

Les titres en IGF-1 sont mesurés sur le sérum obtenu à partir du sang des souris sacrifiées à J56. La mesure est effectuée sur du sérum dilué (1:25) en

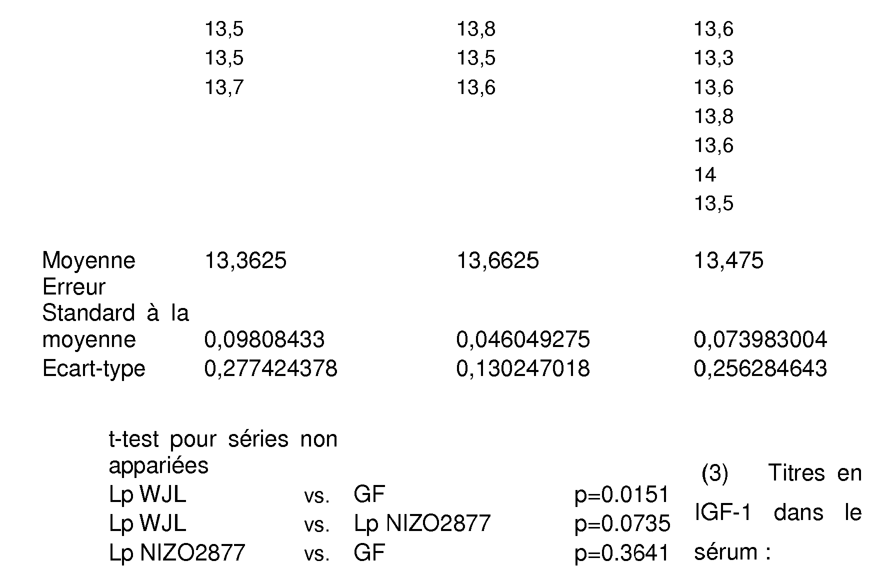

utilisant le kit ELISA Ready-Set-Go (eBioscience, USA), en suivant les instructions du fabricant.

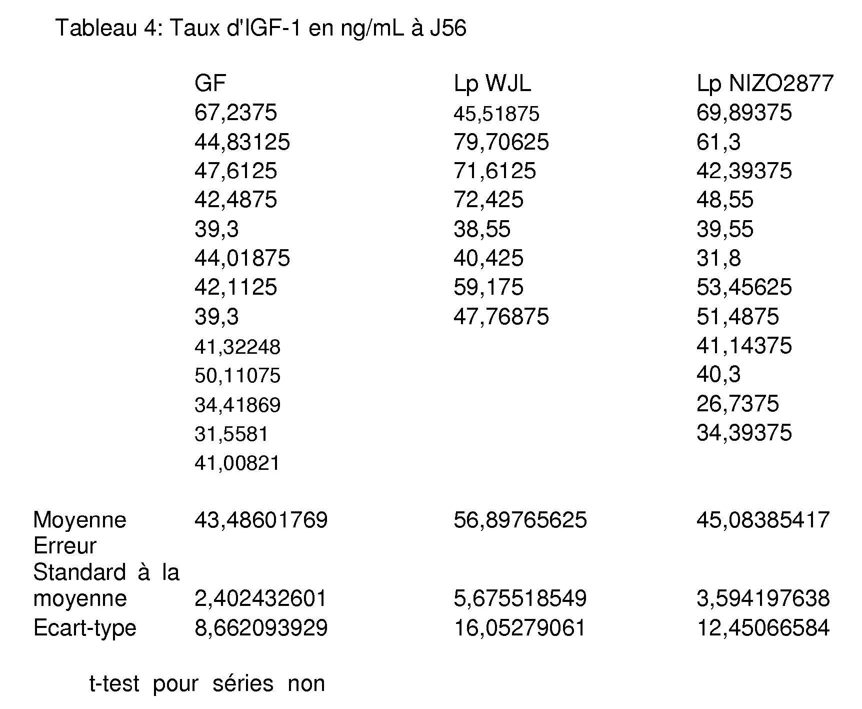

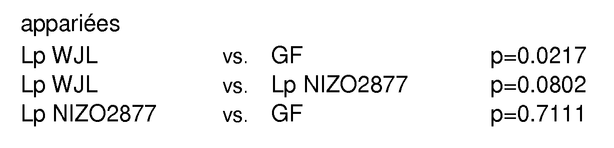

Les résultats illustrent un effet "marqué" de la souche *L. plantarum WJL* sur la croissance linéaire (valeur moyenne supérieure et valeur de p <0.05 par rapport à la condition axénique) et un effet "intermédiaire" de la souche *L. plantarum NIZO2877* (valeur moyenne supérieure et valeur de p<0.05 par rapport à la condition axénique et valeur moyenne inférieure et valeur p<0.05 par rapport à la condition *L. plantarum WJL*). L'effet de la souche *L. plantarum WJL* est confirmé avec les paramètres secondaires que sont la prise de poids, le taux d'IGF-1 et la longueur des fémurs par rapport à la condition axénique (valeur moyenne supérieure et valeur de p<0,05). Toutefois l'effet intermédiaire de la souche *L. plantarum NIZO2877* n'est pas confirmé sur les paramètres secondaires de l'étude. Les paramètres secondaires ne peuvent donc pas être utilisés pour identifier l'effet quantitatif de la souche testée, seul le paramètre principal (croissance linéaire) le permet.

L'ensemble de ces résultats démontre par la preuve scientifique l'effet promoteur de croissance juvénile de certaines souches de *Lactobacilles* et exemplifient un effet "marqué" ou un effet "modéré" de certaines souches sur la croissance linéaire. Un effet "fort" sera obtenu avec certaines souches, cet effet fort correspondant à une valeur moyenne de croissance linéaire supérieure et une valeur de p<0,05 par rapport à la condition *L. plantarum WJL.*

Des souches sont disponibles à l'ATCC, à l'Institut Pasteur de Paris, à l'Institut Pasteur de Lille ou publié dans la littérature scientifique et disponible auprès des chercheurs référents des publications mentionnées :

| | Collection publique et/ou publication avec séquence du génome |
|---|---|
| *Lactobacillus plantarum WJL* | Kim et al., Genome Announc. 21 novembre 2013, 1(6).Pil : e00937-13 |
| *Lactobacillus plantarum NIZO2877* | NIZO (2877) |
| *Lactobacillus casei ATCC 393* | ATCC (393) |
| *Lactobacillus fermentum ATCC 9338* | ATCC (9338) |
| *Lactobacillus fermentum KLD* | Institut Pasteur de Lille (A5.20) |
| *Lactobacillus fermentum LMG* | Institut Pasteur de Lille (A5.16) |
| *Lactobacillus paracasei ATCC 25302* | ATCC (25302) |
| *Lactobacillus paracasei BL23* | Institut Pasteur de Lille (A3.6) et Mazé et al., J. Bacteriol., Mai 2010; 192(10) :2647-8 |
| *Lactobacillus paracasei Shirota* | |
| *Lactobacillus delbrueckii spp.bulgaricus* | Institut Pasteur de Lille (A3.5) |
| *Lactobacillus casei L919* | ATCC (11842) et van de Guchte M, et al., Proc.Natl Acad Sci USA 13 juin 2006 |
| *Lactobacillus rhamnosus L900* | Koryszewska-Baginska A et al., Genome Announc, 26 septembre 2013 |
| *Lactobacillus rhamnosus L908* | Aleksandrzak-Piekarczyk T et al. Genome Announc , 15 août 2013 |
| *Lactobacillus rhamnosus GG* | Koryszewska-Baginska A et al. Genome Announc, 20 février 2014 |
| *Lactobacillus plantarum G821* | ATCC (53103) et Kankainen M et al., Proc Natl Acad Sci USA, 6 octobre 2009 CNCM I-4979 |

De même, l'homme du métier pourra se référer à ces divers documents et aux dépôts de souches commerciales auxquels il est fait référence ici.

## Revendications

1. Utilisation d'une composition probiotique comprenant une souche *Lactobacillus plantarum* WJL, à tropisme intestinal, pour favoriser la croissance juvénile des animaux vertébrés d'élevage en nutrition d'élevage conventionnelle, avec stimulation de la croissance linéaire et/ou du taux d'IGF-1.

2. Utilisation d'une composition probiotique selon la revendication 1 dans laquelle la souche *Lactobacillus plantarum WJL* répond positivement au test suivant :
- à partir d'une même lignée de souris on établit une lignée de souris parentales axéniques et une lignée de souris parentales monoxéniques (associées à la bactérie à tester), et l'on produit des juvéniles qui sont élevées avec les parents en milieu nutritionnel conventionnel comprenant environ 40% d'hydrates de carbone, environ 25% de protéines et environ 9% de lipides jusqu'à leur sevrage (J21)
- à J21 : on dispose de 8 juvéniles sevrées issues de chacune de ces deux lignées, formant le groupe monoxénique et le groupe axénique, et on les élève en régime nutritionnel conventionnel comprenant environ 40% d'hydrates de carbone, environ 25% de protéines et environ 9% de lipides ;
- à J 56 : on détermine la moyenne des tailles des souris pour chaque groupe considéré par la mesure de l'extrémité du museau à la base de la queue de chaque individu, ou l'on prélève les fémurs des individus de chaque groupe et l'on mesure ces derniers ;
- la souche de bactérie lactique étant considérée comme répondant positivement au test si la taille moyenne des individus ou la longueur moyenne des fémurs du groupe monoxénique est supérieur à la taille moyenne des individus ou à l longueur moyenne des fémurs, du groupe axénique, avec une valeur de p inférieure à 0,05 dans le test statistique de Tukey.

3. Utilisation d'une composition probiotique selon l'une quelconque des revendications 1 ou 2, dans laquelle la souche *Lactobacillus plantarum WJL* répond positivement au test suivant :
- à partir d'une même lignée de souris axéniques on établit une lignée de souris parentales axéniques et une lignée de souris parentales monoxéniques associées à la bactérie à tester, et l'on produit des juvéniles qui sont élevées avec les parents en milieu nutritionnel conventionnel comprenant environ 40% d'hydrates de carbone, environ 25% de protéines et environ 9% de lipides jusqu'à leur sevrage (J21),
- à J21 : on dispose de 8 juvéniles sevrées issues de chacune de ces deux lignées, formant le groupe monoxénique et le groupe axénique, et on les élève en régime nutritionnel conventionnel comprenant environ 40% d'hydrates de carbone, environ 25% de protéines et environ 9% de lipides ;
- à J 56 : on prélève du sang des juvéniles de chaque groupe et on détermine le taux sérique moyen d'IGF-1 pour chaque groupe ;
- la souche de bactérie lactique étant considérée comme répondant positivement au test si le taux sérique moyen d'IGF-1 du groupe monoxénique est supérieur au taux sérique moyen du groupe axénique avec une valeur de p inférieure à 0,05 dans le test statistique de Tukey.

4. Utilisation d'une composition probiotique selon l'une quelconque des revendications précédentes, contenant d'environ 10⁵ à environ 10¹² CFU de bactérie lactique, par gramme de composition.

5. Méthode de criblage de bactéries capables de favoriser la croissance juvénile chez un animal vertébré d'élevage en nutrition d'élevage conventionnelle, dans laquelle la souche de bactérie est soumise au test suivant :
- on dispose de juvéniles issues de deux lignées issues d'une même souche de souris, à savoir une lignée de souris axéniques et une lignée de souris monoxéniques,
- on les élève en régime nutritionnel conventionnel comprenant environ 40% d'hydrates de carbone, environ 25% de protéines et environ 9% de lipides;
- au terme d'une période d'élevage appropriée, on détermine la moyenne des valeurs d'un ou plusieurs paramètres de chaque groupe, ces paramètres étant le taux d'IGF-1 sérique ou la croissance linéaire,
- la souche de bactérie lactique est considérée comme répondant positivement au test si la valeur moyenne du paramètre mesuré dans le groupe monoxénique est supérieure à la valeur moyenne du paramètre mesuré dans le groupe axénique avec une valeur de p inférieure à 0,05 dans le test statistique Tukey.

6. Méthode selon la revendication 5, dans laquelle dans ledit test :
- à J21 : on dispose de 8 juvéniles sevrées issues de chacune de ces deux lignées, formant le groupe monoxénique et le groupe axénique, et on les élève en régime nutritionnel conventionnel,
- à J 56 : on détermine la moyenne des tailles des souris pour un groupe considéré par la mesure de l'extrémité du museau à la base de la queue de chaque individu ; ou de la longueur des fémurs prélevés après sacrifice des individus,
- la souche de bactérie lactique étant considérée comme répondant positivement au test si la taille moyenne des individus et/ou la longueur moyenne des fémurs du groupe monoxénique, est supérieure, respectivement, à la taille moyenne des individus et/ou à la longueur moyenne des fémurs du groupe axénique, avec une valeur de p inférieure à 0,05, dans le test statistique de Tukey.

## Patentansprüche

1. Verwendung einer probiotischen Zusammensetzung, die einen *Lactobacillus plantarum* WJL-Stamm mit intestinalem Tropismus umfasst, zur Förderung des juvenilen Wachstums von mit herkömmlicher Fütterung in der Tierhaltung gezüchteten Wirbeltieren mit Stimulation des linearen Wachstums und/oder des IGF-1-Spiegels.

2. Verwendung einer probiotischen Zusammensetzung nach Anspruch 1, wobei der *Lactobacillus plantarum* WJL-Stamm positiv auf den folgenden Test reagiert:
- Etablieren, ausgehend von einer selben Mauslinie, einer axenischen Elternmauslinie und einer monoxenischen (mit dem zu testenden Bakterium verbundenen) Elternmauslinie und Erzeugen von Jungtieren, die bis zu ihrer Entwöhnung (Tag 21) zusammen mit den Eltern mit einer herkömmlichen Ernährungsweise aufgezogen werden, die etwa 40 % Kohlenhydrate, etwa 25 % Proteine und etwa 9 % Lipide umfasst
- am Tag 21: Bereitstellen von 8 entwöhnten Jungtieren, die aus jeder dieser beiden Linien stammen, welche die monoxenische Gruppe und die axenische Gruppe bilden, und deren Aufziehen mit einer herkömmlichen Ernährungsweise, die etwa 40 % Kohlenhydrate, etwa 25 % Proteine und etwa 9 % Lipide umfasst;
- am Tag 56: Bestimmen des Durchschnitts der Größen der Mäuse für jede betrachtete Gruppe durch Messen von der Schnauzenspitze bis zum Schwanzansatz jedes einzelnen Individuums oder Entnehmen der Oberschenkelknochen der Individuen jeder Gruppe und Messen dieser letzteren;
- wobei der Milchsäurebakterienstamm als positiv auf den Test reagierend betrachtet wird, wenn die durchschnittliche Größe der Individuen oder die durchschnittliche Länge der Oberschenkelknochen der monoxenischen Gruppe größer ist als die durchschnittliche Größe der Individuen oder die durchschnittliche Länge der Oberschenkelknochen der axenischen Gruppe, mit einem p-Wert von kleiner als 0,05 im statistischen Tukey-Test.

3. Verwendung einer probiotischen Zusammensetzung nach einem der Ansprüche 1 oder 2, wobei der *Lactobacillus plantarum* WJL-Stamm positiv auf den folgenden Test reagiert:
- Etablieren, ausgehend von einer selben axenischen Mauslinie, einer axenischen Elternmauslinie und einer monoxenischen, mit dem zu testenden Bakterium verbundenen Elternmauslinie und Erzeugen von Jungtieren, die bis zu ihrer Entwöhnung (Tag 21) zusammen mit den Eltern mit einer herkömmlichen Ernährungsweise aufgezogen werden, die etwa 40 % Kohlenhydrate, etwa 25 % Proteine und etwa 9 % Lipide umfasst,
- am Tag 21: Bereitstellen von 8 entwöhnten Jungtieren, die aus jeder dieser beiden Linien stammen, welche die monoxenische Gruppe und die axenische Gruppe bilden, und deren Aufziehen mit einer herkömmlichen Ernährungsweise, die etwa 40 % Kohlenhydrate, etwa 25 % Proteine und etwa 9 % Lipide umfasst;
- am Tag 56: Entnehmen von Blut von den Jungtieren jeder Gruppe und Bestimmen des durchschnittlichen Serumspiegels von IGF-1 für jede Gruppe;
- wobei der Milchsäurebakterienstamm als positiv auf den Test reagierend betrachtet wird, wenn der durchschnittliche Serumspiegel von IGF-1 der monoxenischen Gruppe höher ist als der durchschnittliche Serumspiegel der axenischen Gruppe, mit einem p-Wert von kleiner als 0,05 im statistischen Tukey-Test.

4. Verwendung einer probiotischen Zusammensetzung nach einem der vorhergehenden Ansprüche, die pro Gramm Zusammensetzung zwischen etwa 10⁵ und etwa 10¹² CFU Milchsäurebakterien enthält.

5. Verfahren zum Screening auf Bakterien, die in der Lage sind, das juvenile Wachstum eines mit herkömmlicher Fütterung in der Tierhaltung gezüchteten Wirbeltiers zu fördern, wobei der Bakterienstamm dem folgenden Test unterzogen wird:
- Bereitstellen von Jungtieren, die aus zwei Linien stammen, die aus einem selben Mausstamm stammen, nämlich einer axenischen Mauslinie und einer monoxenischen Mauslinie,
- deren Aufziehen mit einer herkömmlichen Ernährungsweise, die etwa 40 % Kohlenhydrate, etwa 25 % Proteine und etwa 9 % Lipide umfasst;
- am Ende einer angemessenen Aufzuchtperiode, Bestimmen des Durchschnitts der Werte eines oder mehrerer Parameter jeder Gruppe, wobei diese Parameter der IGF-1-Spiegel des Serums oder das lineare Wachstum sind,
- wobei der Milchsäurebakterienstamm als positiv auf den Test reagierend betrachtet wird, wenn der in der monoxenischen Gruppe gemessene Durchschnittswert des Parameters höher ist als der Durchschnittswert des in der axenischen Gruppe gemessenen Parameters, mit einem p-Wert von kleiner als 0,05 im statistischen Tukey-Test.

6. Verfahren nach Anspruch 5, wobei der Test umfasst:
- am Tag 21: Bereitstellen von 8 entwöhnten Jungtieren, die aus jeder dieser beiden Linien stammen, welche die monoxenische Gruppe und die axenische Gruppe bilden, und deren Aufziehen mit einer herkömmlichen Ernährungsweise,
- am Tag 56: Bestimmen des Durchschnitts der Größen der Mäuse für eine betrachtete Gruppe durch Messen von der Schnauzenspitze bis zum Schwanzansatz jedes Individuums; oder der Länge der nach der Tötung der Individuen entnommenen Oberschenkelknochen,
- wobei der Milchsäurebakterienstamm als positiv auf den Test reagierend betrachtet wird, wenn die durchschnittliche Größe der Individuen und/oder die durchschnittliche Länge der Oberschenkelknochen der monoxenischen Gruppe jeweils größer ist als die durchschnittliche Größe der Individuen und/oder die durchschnittliche Länge der Oberschenkelknochen der axenischen Gruppe, mit einem p-Wert von kleiner als 0,05 im statistischen Tukey-Test.

## Claims

1. Use of a probiotic composition comprising a strain of *Lactobacillus plantarum* WJL, with intestinal tropism, in promoting juvenile growth in vertebrate livestock fed a conventional rearing diet, with stimulation of linear growth and/or of IGF-1 level.

2. The use of a probiotic composition according to claim 1, wherein the *Lactobacillus plantarum* WJL strain responds positively to the following test:
- from the same mouse line, a line of axenic parent mice and a line of monoxenic parent mice (associated with the bacterium to be tested) are established, and juveniles are produced which are raised with the parents on a conventional diet comprising about 40% carbohydrates, about 25% proteins and about 9% lipids until they are weaned (day 21);
- on day 21: 8 weaned juveniles from each of these two lines are available, forming the monoxenic group and the axenic group, and they are raised on a conventional diet comprising about 40% carbohydrates, about 25% proteins and about 9% lipids;
- on day 56: the mean size of the mice is determined for each group in question by measuring from the tip of the nose to the base of the tail of each individual, or the femurs are removed from the individuals of each group and measured;
- the lactic acid bacteria strain being considered to respond positively to the test if the mean size of the individuals or the mean length of the femurs of the monoxenic group is greater than the mean size of the individuals or the mean length of the femurs of the axenic group, with a p-value of less than 0.05 in Tukey's statistical test.

3. The use of a probiotic composition according to any one of claims 1 or 2, wherein the *Lactobacillus plantarum* WJL strain responds positively to the following test:
- from the same line of axenic mice, a line of axenic parent mice and a line of monoxenic parent mice (associated with the bacterium to be tested) are established, and juveniles are produced which are raised with the parents on a conventional diet comprising about 40% carbohydrates, about 25% proteins and about 9% lipids until they are weaned (day 21);
- on day 21: 8 weaned juveniles from each of these two lines are available, forming the monoxenic group and the axenic group, and they are raised on a conventional diet comprising about 40% carbohydrates, about 25% proteins and about 9% lipids;
- on day 56: blood is drawn from the juveniles of each group and the mean serum IGF-1 level is determined for each group;
- the lactic acid bacteria strain being considered to respond positively to the test if the mean serum IGF-1 level of the monoxenic group is higher than the mean serum level of the axenic group with a p-value of less than 0.05 in Tukey's statistical test.

4. The use of a probiotic composition according to any one of the preceding claims, containing about 10⁵ to about 10¹² CFU of lactic acid bacterium, per gram of composition.

5. A method for screening bacteria capable of promoting juvenile growth in a vertebrate livestock animal fed a conventional rearing diet, wherein the bacterial strain is subjected to the following test:
- juveniles are provided from two lines derived from the same mouse strain, namely a line of axenic mice and a line of monoxenic mice;
- they are raised on a conventional diet comprising about 40% carbohydrates, about 25% proteins and about 9% lipids;
- at the end of a suitable rearing period, the mean value of one or more parameters is determined for each group, these parameters being the serum IGF-1 level or linear growth;
- the lactic acid bacteria strain is considered to respond positively to the test if the mean value of the parameter measured in the monoxenic group is higher than the mean value of the parameter measured in the axenic group with a p-value of less than 0.05 in Tukey's statistical test.

6. The method according to claim 5, wherein in said test:
- on day 21: 8 weaned juveniles from each of these two lines are available, forming the monoxenic group and the axenic group, and they are raised on a conventional diet,
- on day 56: the mean size of the mice is determined for a group in question by measuring from the tip of the nose to the base of the tail of each individual; or of the length of femurs removed from the individuals after sacrifice,
- the lactic acid bacteria strain being considered to respond positively to the test if the mean size of the individuals and/or the mean length of the femurs of the monoxenic group is greater than the mean size of the individuals and/or the mean length of the femurs of the axenic group, respectively, with a p-value of less than 0.05 in Tukey's statistical test.
